# EUROPÄISCHE PATENTANMELDUNG

(11) **EP 1 844 808 A1**
(43) Veröffentlichungstag der Anmeldung: **17.10.2007**
(21) Anmeldenummer: 06405162.6
(22) Anmeldetag: 13.04.2006
(51) Int. Cl.: A61M 15/00

(54) **Medikamenten-Ausgabevorrichtung**

(71) Anmelder: Boehringer Ingelheim Pharma GmbH & Co. KG, 55216 Ingelheim am Rhein (DE)
(72) Erfinder: Wachtel, Herbert, 55218 Ingelheim am Rhein (DE); Geser, Johannes, 55218 Ingelheim am Rhein (DE); Metzger, Burkhard, 55218 Ingelheim am Rhein (DE); Spallek, Michael, 55218 Ingelheim am Rhein (DE); Krueger, Michael, 55218 Ingelheim am Rhein (DE); Kunze, Hubert, 44227 Dortmund (DE); Moser, Achim, 65843 Sulzbach (DE); Mock, Elmar, 2013 Colombier (CH); Lanci, Antonio, 3006 Bern (CH); Klopfenstein, André, 2520 La Neuveville (CH)
(74) Vertreter: Frei Patent Attorneys

(57) **Zusammenfassung**

Die Erfindung bezieht sich auf eine Medikamenten-Ausgabevorrichtung, insbesondere einen Mehrdosispulverinhalator, zur Ausgabe von Medikamenten- Einzeldosen. Dabei liegen eine Vielzahl von Medikamentenkammern (4,5) in einem Medikamentenmagazin in der Form einer Endlosschlaufe vor. Die Medikamentenkammern bilden Gruppen, derart dass ein Mundstück (2) verschiedene Gruppen durch einen im wesentlichen vollständigen Umgang des Medikamentenmagazins erreicht. Zwischen zwei aufeinanderfolgenden Entnahmnepositionen können somit mehrere Medikamentenkammern (4,5), typischerweise eine, zwei oder drei, angeordnet sein. Damit ist es möglich eine sehr hohe Dichte an Medikamentenkammern und damit eine Inhalationsvorrichtung zu schaffen, welche sehr handlich gestaltet werden kann, auch bei grosser Anzahl von Einzeldosen.

## Beschreibung

Die Erfindung liegt auf dem Gebiet von Medikamenten-Ausgabevorrichtungen mit einem Mehrfachmagazin, insbesondere von Inhalationsgeräten für pulverförmige Medikamente und betrifft eine Medikamenten-Ausgabevorrichtung gemäss dem Oberbegriff des unabhängigen Patentanspruchs.

Aus dem US Patent Nr. 4,627,432 ist ein Inhalationsgerät bekannt, in welchem eine Blisterpackung mit kreisförmig angeordneten Blistern durch eine Rotation weiter transportiert wird. Dabei werden die in der Blisterpackung eingebrachten Blister nacheinander in eine Inhalationsposition gebracht. Die einzelnen Blister werden zum Öffnen über einen Hebel angestochen. Dies macht die Vorrichtung relativ gross, da ein gewisser Hub für das Anstechen notwendig ist. Soll ein Öffnungsmechanismus bei bzw. in einem Mundstück angeordnet werden, wie beispielsweise im Dokument US 5,590,645 beschrieben, so ist für das Weitertransportieren und Öffnen eine relativ hohe Kraft nötig. Diese Kraft wird vorzugsweise auf einen längeren Weg verteilt, um die Bedienung des Gerätes zu erleichtern. Einen weiteren Weg bzw. eine grössere auszuführende Bewegung auf eine seitliche Bewegung eines Mundstücks zu übertragen, hätte jedoch den Nachteil, dass die einzelnen Blister in der Blisterpackung entsprechend weit voneinander entfernt sein müssten. Dies ist in Bezug auf Multidose-Inhalatoren mit einer möglichst hohen Einzeldosenzahl nachteilig und dem Bedürfnis für kleine handliche Geräte abträglich.

Aufgabe der Erfindung ist es, eine Medikamenten-Ausgabevorrichtung zu schaffen, mit welcher eine genügend grosse Bewegung zum Positionieren und Öffnen einer Medikamentenkammer zur Verfügung gestellt wird, bei gleichzeitig dichter Packung von Medikamentenkammern in einem Medikamentenmagazin, welche insbesondere auch für Medikamentenmagazine mit ringförmig angeordneten Medikamentenkammem geeignet ist.

Die Aufgabe wird durch die Medikamenten-Ausgabevorrichung gelöst, wie sie in den Ansprüchen definiert ist.

Die Medikamenten-Ausgabevorrichtung, vorzugsweise ein Mehrdosispulverinhalator, zur Ausgabe von Medikamenten-Einzeldosen, weist eine Vielzahl von Medikamentenkammern in einem Medikamentenmagazin auf, wobei das Magazin bzw. die darin eingebrachten Kammern in der Form einer Endlosschlaufe vorliegen. Das Medikamentenmagazin und ein Mundstück, durch welches ein Medikament von einem Patienten eingenommen, beispielsweise inhaliert werden kann, sind relativ zueinander verschiebbar, so dass sämtliche Medikamentenkammern und das Mundstück nacheinander miteinander in Übereinstimmung bringbar sind. Dabei bilden die Medikamentenkammern jeweils Gruppen, derart, dass das Mundstück verschiedene Gruppen durch einen im wesentlichen vollständigen Umgang des Medikamentenmagazins, d.h. nach einem im wesentlichen vollständigen Umgang der Schlaufe erreicht.

Ein in wesentlichen vollständiger Umgang bedeutet, dass ein Mundstück, welches sich zur Entnahme in einer anfänglichen Medikamentenkammerposition x befindet, sich nach einem im wesentlichen vollständigen Umgang vorzugsweise in einer Medikamentenkammerposition x + 1 oder x - 1 befindet. Da ein Mundstück und ein Medikamentenmagazin oder ein Gehäuse relativ zueinander verschiebbar angeordnet sind, kann sich der Umgang auf das Mundstück um das Magazin oder das Gehäuse oder auch auf eine fast vollständige Umdrehung um 360° bzw. um wenig mehr als 360° eines Medikamentenmagazins in Bezug auf ein Mundstück oder das Gehäuse beziehen.

Die erfindungsgemässe Medikamenten-Ausgabevorrichtung erlaubt nun, dass zwei aufeinanderfolgende Entnahmepositionen nicht gleich zwei nebeneinander angeordneten Kammerpositionen entsprechen. Zwischen zwei aufeinanderfolgenden Entnahmepositionen können mehrere Medikamentenkammern, typischerweise eine, zwei oder drei, angeordnet sein.

Ein zum Öffnen einer Medikamentenkammer benötigter Vorschub kann nun im wesentlichen so lang sein, wie erforderlich, ohne dass ein dazwischenliegender Bereich für den Gebrauch als Speicherplatz für Medikamentenkammern entfällt.

Dies erlaubt eine sehr hohe Medikamentenkammerdichte und damit die Schaffung eines Inhalators, welcher auch ohne Wechsel eines Medikamentenmagazins, Medikamenten-Einzeldosen für mehrere Wochen oder Monate, z. B. 30-60 Einzeldosen, beinhaltet. Dies ist bei gleichbleibender, gegebenenfalls auch geringerer Inhalatorgrösse möglich. Das Überspringen einzelner Medikamentenkammern bei einem bestimmten Umgang stellt den erforderlichen Weg zur Verfügung, der beispielsweise für einen Hub für einen Öffnungsmechanismus für eine Medikamentenkammer, z.B. mittels Stechen, erforderlich ist. Es ist aber auch möglich, die teilweise hohe Kraft, die zum Transport eines Magazins und zum Öffnen einer Kammer notwendig ist, in einen längeren Weg umzusetzen, ohne dabei Raum für Medikamentenkammern zu verlieren. Dies ist insbesondere von Vorteil bei scheibenförmigen Inhalatoren, in welchen ein Medikamentenmagazin als Ringscheibe vorliegt und die einzelnen Medikamentenkammern kreisförmig in diesem Magazin angeordnet sind. Soll nun die Transport- und Öffnungsbewegung in eine Bewegung beispielsweise eines Mundstücks entlang einem Aussenumfang der Ringscheibe integriert sein, so ist ein langer Vorschub des Mundstücks und gleichzeitig eine Anordnung von Medikamentenkammern möglich, welche dichter beieinander liegt, als eine entsprechende Vorschubdistanz.

Unterschiedliche Öffnungsmechanismen, wie beispielsweise Anstechen, Peelen oder Schaben, können damit direkt in die Bewegung eines Mundstücks integriert bzw. mit der Bewegung des Mundstücks kombiniert werden.

In einer bevorzugten Ausführungsform sind Mundstück und Medikamentenmagazin nur in einer Richtung relativ zueinander verschiebbar. Auch sind die Medikamentenkammern vorzugsweise äquidistant im Medikamentenmagazin angeordnet und ein Vorschub weist jeweils dieselbe Länge auf.

Ein für die Medikamenten-Ausgabevorrichtung geeignetes Medikamentenmagazin bzw. die darin eingebrachten Medikamentenkammern liegen in einer Endlosschlaufe vor. Dies können im wesentlichen jegliche Arten von zyklischen Anordnungen von Medikamentenkammern sein. Typische Beispiele sind Ringscheiben mit kreisförmig eingebrachten Medikamentenmagazinen oder auch endlos Blisterbänder, beispielsweise in runder oder ovaler Anordnung.

Die Medikamenten-Ausgabevorrichtung weist vorzugsweise eine Anzeige zum Anzeigen in welcher Medikamentenkammer- oder Entnahmeposition sich das Mundstück befindet auf. Es ist auch möglich in einer solchen Anzeige eine Angabe über eine Gruppe von Medikamentenkammern zu machen. Dies ist insbesondere bei Vorrichtungen bzw. Medikamentenmagazinen von Vorteil, welche beispielsweise Gruppen von Medikamentenkammern aufweisen, in welchen eine sich ändernde Dosierung oder Zusammensetzung eines Medikaments befindet.

Die Anzahl der Medikamentenkammern oder Medikamenteneinzeldosen für die Vorrichtung liegt vorzugsweise in einem Bereich von 1 bis 100 oder bis 200 Einzeldosen, bevorzugt in einem Bereich von 1-60, beispielsweise zwischen 7-180 oder 14-150, z. B. 30-120, 45-100, 30, 90, 60, 120. Für Inhalationsgeräte liegt eine Maximalanzahl von Einzeldosen aus Handlichkeits- und Therapie-Gründen vorzugsweise bei 60.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Besonders bevorzugt sind in diesem Zusammenhang Arzneimittel, die ausgewählt sind aus der Gruppe bestehend aus Anticholinergika, Betamimetika, Steroiden, Phosphodiesterase IV-inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika. Bevorzugt werden Anticholinergika-haltige Wirkstoffe eingesetzt, als Monopräparate oder in Form von Kombinationspräparaten.

Im einzelnen seien als Beispiele für die wirksamen Bestandteile oder deren Salze genannt:

Zur Anwendung gelangende Anticholinergika sind bevorzugt ausgewählt aus der Gruppe bestehend aus Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinestermethobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethylxanthen-9-earbonsäuretropenolester Methobromid, 9-Difluormethyl-xanthen-9-carbonsäuretropenolester -Methobromid und 9-Hydroxymethyl-xanthen-9-carbonsäurescopinester -Methobromid, gegebenenfalls in Form ihrer Racemate, Enantiomere oder Diastereomere und gegebenenfalls in Form ihrer Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacaterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethylphenyl)-ethylamino]-hexyloxy}-butyl)-benzolsulfonamid, 5-[2-(5,6-Diethyl-indan-2-ylamino)-1-hydroxy-ethyl]-8-hydroxy-1*H*-quinolin-2-on, 4-hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol , 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol , 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol , 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11 β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-Inhibitoren sind bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methylisothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, C1-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2- hydroxy-2-propyl)phenyl)thio)methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VLTF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluorphenyl)amino]-6-[2-((S)-6-methyl-2-oxo-morpholin-4-yl)-ethoxy]-7-methoxychinazolin, 4-[(3-Chlor-4-fluorphenyl)amino]-6-({4-[N-(2-methoxy-ethyl)-N-methyl-amino]-1-oxo-2-buten-1-yl}amino)-7-cydopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6- {1-[(morpholin-4-yl)carbonyl]-piperidin-4-yloxy}-7-methoxychinazolin, 4-[(3-Chlor-4-fluor-phenyl)amino]-6-(piperidin-3-yloxy)-7-methoxychinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6- {trans-4-[(morpholin-4-yl)carbonylamino]-cyclohexan-1-yloxy}-7-methoxychinazolin,

4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxychinazolin, 4-[(3-Chlor-4-fluor-phenyl)amino]-6-(trans-4-ethansulfonylaminocyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4yloxy]-7-methoxy-chinazolin, 4-[(3-Chlor-4-fluor-phenyl)amino]-6-(cis-4-{N-[(piperidm-1-yl)carbonyl]-N-methyl-ammo}-cyclohexan-1-yloxy)-7-methoxychinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluorphenyl)amino]-6-{1-[2-(2-oxopyrrolidin-1-yl)ethyl]-piperidin-4-yloxy}-7-methoxychinazolin, 4-[(3-Ethinyl-phenyl)amino]-6-(1-acetyl-piperidin-4-yloxy)-7-methoxychinazolin, 4-[(3-Ethinyl-phenyl)amino]-6-(1-methyl-piperidin-4-yloxy)-7-methoxychinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)cazbonyl]-piperidin-4-yloxy}-7-methoxy-chinazolin, 4-[(3-Chlor-4-fluor-phenyl)amino]-6-(1-ethylpiperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluorphenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonylpiperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluorphenyl)amino]-6- {1-[(2-methoxyethyl)carbonyl]-piperidin-4-yloxy)-7-methoxychinazolin, gegebenenfalls in Form ihrer Racemate, Enantiomere oder Diastereomere gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Antiallergika: Dinatriumcromoglicat, Nedocromil.

Als Derivate der Mutterkornalkaloide: Dihydroergotamin, Ergotamin.

Für die Inhalation kommen Arzneimittel, Arzneimittelformulierungen und - mischungen mit den o.g. Wirkstoffen in Betracht, sowie deren Salze, Ester sowie die Kombination dieser Wirkstoffe, Salze und Ester.

Im Folgenden ist die Erfindung anhand von vereinfachten, schematisch dargestellten Beispielen gezeigt. Dabei zeigt
- Fig. 1: ein Medikamentenmagazin mit zwei Gruppen von Medikamentenkammern
- Fig. 2: eine Medikamenten-Ausgabevorrichtung
- Fig. 3: eine weitere Medikamenten-Ausgabevorrichtung

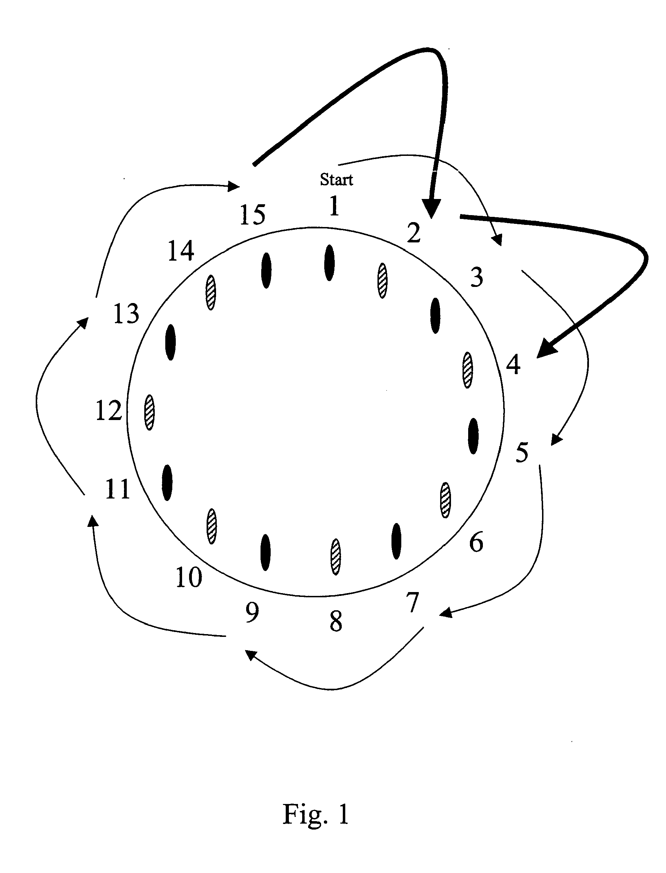

In **Figur 1** ist das Prinzip der erfindungsgemässen Medikamenten-Ausgabevorrichtung anhand von kreisförmig, äquidistant angeordneten Medikamentenkammern gezeigt. Die insgesamt 15 Medikamentenkammern sind in zwei Gruppen unterteilt. Die erste Gruppe von Medikamentenkammern sind in der Figur als gefüllte Ovale, die zweite Gruppe als gestrichelte Ovale dargestellt. Nachdem bei eine Medikamentenkammer Nr.1 gestartet wird und diese verbraucht ist, wird jeweils eine übernächste Kammer, Nr. 3, dann Nr.5 etc. angefahren, solange, bis sämtliche Kammern der Gruppe 1 abgefahren, d.h. verbraucht sind. Die Position der letzten Kammer der Gruppe 1, hier Nr. 15, liegt benachbart zur Kammer Nr. 1, um eine halbe Vorschubdistanz entfernt. Mit derselben Vorschubdistanz wie bisher, z.B. durch eine Vorwärtsbewegung eines Mundsstücks oder eine entsprechende Bewegung des Magazin bzw. Gehäuses, wird die erste Kammer der zweiten Gruppe, Nr. 2, angefahren. Nun werden sämtliche Kammern der Gruppe 2 nacheinander angefahren, wobei jeweils die bereits verbrauchten Kammern der Gruppe 1 übersprungen werden.

Eine Medikamentenanzeige, welche anzeigt, die wievielte Kammer gerade benutzt wird, bzw. wieviele Kammern noch im Magazin verbleiben, ist vorzugsweise in der Reihenfolge numeriert, wie die Kammern angefahren werden.

In diesem Beispiels wird jeweils eine Medikamentenkammer übersprungen. Nach einer knappen Umdrehung, welche einer ganzen Umdrehung minus einer halben Vorschubdistanz entspricht, wird von einer ersten Gruppe von Medikamentenkammern in eine zweite Gruppe übergegangen.

An diesem Beispiel ist klar ersichtlich, dass je nach Vorschubdistanz und Anordnungsdichte der Medikamentenmagazine auch ein Überspringen von zwei, drei oder mehreren Medikamentenkammern erforderlich oder vorteilhaft ist. Entsprechend sind die Kammern in drei, vier, oder mehrere Gruppen unterteilt, wobei die letzte Kammer der ersten Gruppe vorzugsweise jeweils um weniger als eine Vorschubdistanz von der ersten Kammer der ersten Gruppe beabstandet ist.

Dieses Beispiel zeigt zudem eine äquidistante Anordnung sämtlicher Medikamentenkammern (aber nicht der einzelnen Gruppen) und einen darauf abgestimmten ebenfalls äquidistanten Vorschub. Eine solche Ausführungsform ist aufgrund der einfachen Geometrie für ein Medikamentenmagazin und aufgrund der kontinuierlichen Bewegung und einer entsprechend einfachen Mechanik für den Vorschub vorteilhaft.

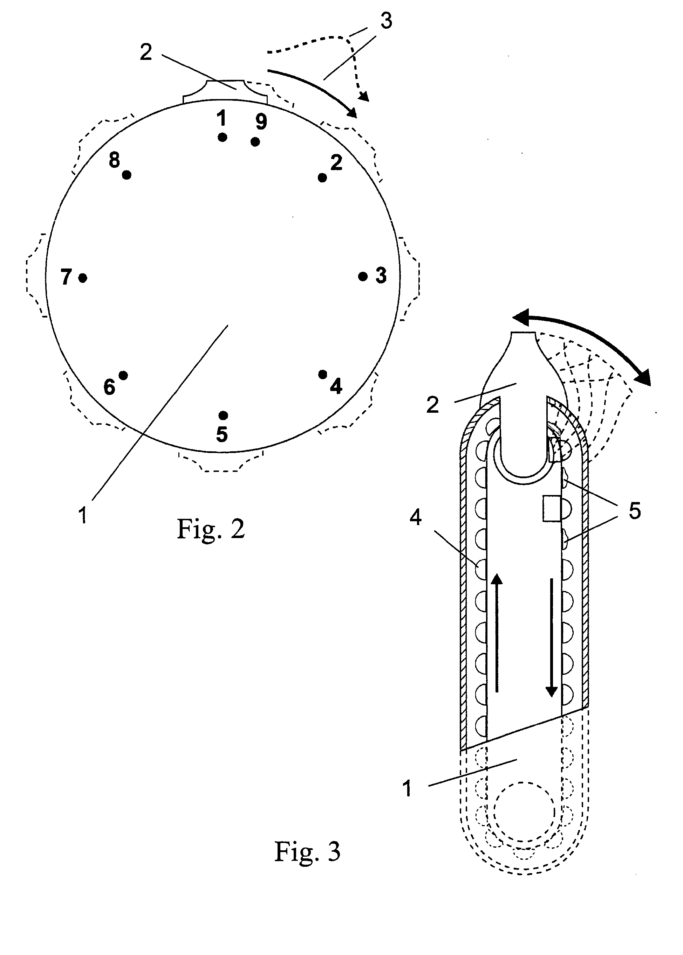

In **Figur 2** ist ein rundes, scheibenförmiges Inhalationsgerät, beispielsweise ein Mehrdosispulverinhalator, mit einem an seinem Aussenumfang angebrachten Mundstück 2 gezeigt. Dabei beziffern die Zahlen 1 bis 9 die Medikamentenkammer- und Entnahmepositionen in der Reihenfolge, wie sie angefahren werden. Dazwischen liegende Kammerpositionen sind bis auf die Position Nr. 9 nicht eingezeichnet. In dieser Ausführungsform wird das Mundstück 2 jeweils um eine bestimmte Vorschubdistanz 3, hier mit Pfeilen eingezeichnet, mit der nächsten Medikamentenkammer in Übereinstimmung gebracht. Der dunklere, radial verlaufende Vorschubpfeil entspricht im wesentlichen der Bewegungsrichtung des Mundstücks, während der gestrichelte, bogenförmig eingezeichnete Pfeil einer Vorschubdistanz eines Mundstück oder einer im Inneren befindlichen Mechanik entspricht, welche beispielsweise aufgrund eines Stechvorganges einen gewissen Hub vor der Positionierung vor einer neuen Medikamentenkammer aufweisen muss.

Zwischen der Position 8 und 9 liegt kein aquidistanter Vorschub. Ein solcher Zusatzvorschub zwischen einer Gruppe und einer nächsten Gruppe kann mit einer entsprechenden Mechanik im Gehäuse 1 vorgenommen und gleichzeitig als Indikator verwendet werden. Ein solcher Indikator kann beispielsweise als zusätzlicher Hinweis zwischen einzelnen zu verabreichenden Indikationen, Dosierungen oder Medikamentenzusammensetzungen dienen, z. B 1. Woche/2. Woche, 1. Monat: niedrigere Dosierung/2. Monat höhere Dosierung oder 1.-10. Tag: erstes Medikament/11.-16.Tag: zweites Medikament.

Unterschiedliche Positionen des Mundstücks 2 in den Medikamentenkammerpositionen 2 bis 9 sind in der Figur gestrichelt gezeichnet.

In **Figur 3** ist eine Medikamenten-Ausgabevorrichtung mit einem raupenförmig eingebrachten endlos Blisterband 4 gezeigt. Über das Mundstück 2 wird das Blisterband weitertransportiert, wobei das Mundstück beweglich, aber im wesentlichen ortsfest am Gehäuse 1 angebracht ist. Auf der einen Seite des Mundstücks ist jeweils ein übernächster Blister als verbrauchte Medikamentenkammer 5 eingezeichnet. Das Medikamentenmagazin bewegt sich bezüglich des Mundstücks und Gehäuses, wobei verschiedene Mundstückpositionen gestrichelt eingezeichnet sind.

## Patentansprüche

1. Medikamenten-Ausgabevorrichtung zur Ausgabe von Medikamenten-Einzeldosen, wobei eine Vielzahl von Medikamentenkammern in einem Medikamentenmagazin in der Form einer Endlosschlaufe vorliegen, und wobei das Medikamentenmagazin und ein Mundstück relativ zueinander verschiebbar angeordnet sind, derart dass sämtliche Medikamentenkammern und das Mundstück nacheinander miteinander in Übereinstimmung bringbar sind, **dadurch gekennzeichnet, dass** die Medikamentenkammern Gruppen bilden, derart, dass das Mundstück verschiedene Gruppen durch einen im wesentlichen vollständigen Umgang des Medikamentenmagazins erreicht, zum Öffnen der Medikamentenkammern einer nächsten Gruppe.

2. Medikamenten-Ausgabevorrichtung nach Anspruch 1, wobei nach einem im wesentlichen vollständigen Umgang des Medikamentenmagazins, das Mundstück in einer Entnahmeposition ist, welche einer Medikamentenkammerposition x + 1 oder x - 1 entspricht, bei einer anfänglichen Medikamentenkammerposition x.

3. Medikamenten-Ausgabevorrichtung nach Anspruch 1 oder 2, wobei Mundstück und Medikamentenmagazin nur in einer Richtung relativ zueinander verschiebbar angeordnet sind.

4. Medikamenten-Ausgabevorrichtung nach einem der Ansprüche 1-3, wobei die Medikamentenkammern äquidistant im Medikamentenmagazin angeordnet sind.

5. Medikamenten-Ausgabevorrichtung nach einem der Ansprüche 2-4, wobei zwischen zwei aufeinanderfolgenden Entnahmepositionen, eine, zwei oder drei Medikamentenkammern angeordnet sind.

6. Medikamenten-Ausgabevorrichtung nach einem der Ansprüche 1-5, wobei das Medikamentenmagazin in einem im wesentlichen scheibenförmigen Gehäuse untergebracht ist und das Mundstück entlang einem Aussenumfang der Scheibe verschiebbar angeordnet ist.

7. Medikamenten-Ausgabevorrichtung nach einem der Ansprüche 1-6, wobei die Medikamentenkammern ringförmig im Medikamentenmagazin angeordnet sind.

8. Medikamenten-Ausgabevorrichtung nach einem der Ansprüche 1-7, wobei die Medikamentenkammern in einem endlos Blisterband angeordnet sind.

9. Medikamenten-Ausgabevorrichtung nach einem der Ansprüche 2-8, mit einer Anzeige zum Anzeigen in welcher Medikamentenkammerposition und/oder Entnahmeposition sich das Mundstück befindet.

10. Medikamenten-Ausgabevorrichtung nach einem der vorhergehenden Ansprüche, wobei bei einem Betätigen des Mundstücks, ein Medikamentenmagazin bezüglich des Gehäuses nicht verschoben wird.

11. Medikamenten-Ausgabevorrichtung nach einem der vorhergehenden Ansprüche, wobei die Vorrichtung ein Mehrdosispulverinhalator ist.

12. Medikamenten-Ausgabevorrichtung nach einem der vorhergehenden Ansprüche, wobei die Vielzahl der Medikamentenkammern maximal 60 beträgt.

13. Medikamenten-Ausgabevorrichtung nach einem der vorhergehenden Ansprüche zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer.
